Europäisches Patentamt

European Patent Office

Office européen des brevets

(19)

(11) Veröffentlichungsnummer: **0 234 378**
**A2**

## (12) EUROPÄISCHE PATENTANMELDUNG

(21) Anmeldenummer: 87101708.3

(22) Anmeldetag: 07.02.87

(51) Int. Cl.⁴: **A61M 3/00**

(30) Priorität: 21.02.86 DE 8604613 U

(43) Veröffentlichungstag der Anmeldung:
02.09.87 Patentblatt 87/36

(84) Benannte Vertragsstaaten:
AT BE CH DE ES FR GB GR IT LI LU NL SE

(71) Anmelder: **Dr. Karl Thomae GmbH**
**Postfach 1755**
**D-7950 Biberach (Riss)(DE)**

(72) Erfinder: **Gläsel, Volker Ingo, Dr. Dipl.-Chem.**
**Hegauweg 7**
**D-7950 Biberach(DE)**
Erfinder: **Landwehr, Frank**
**Berg Nr. 9**
**D-7951 Hochdorf(DE)**

(54) **Kolbenapplikator mit Klemmvorrichtung zur Aufnahme eines Primärpackmittels in Form eines langgestreckten zylindrischen Röhrchens.**

(57) Beschrieben wird ein Kolbenapplikator mit Klemmvorrichtung zur Aufnahme eines Primärpackmittels in Form eines langgestreckten zylindrischen Röhrchens. Der Kolbenapplikator dient zur Verabreichung geringer Mengen von Arzneiflüssigkeiten.

EP 0 234 378 A2

## Kolbenapplikator mit Klemmvorrichtung zur Aufnahme eines Primärpackmittels in Form eines langgestreckten zylindrischen Röhrchens

Die Erfindung betrifft einen Kolbenapplikator mit Klemmvorrichtung zur Aufnahme eines Primärpackmittels in Form eines langgestreckten zylindrischen Röhrchens.

Zur Verabreichung geringer Mengen von Arzneiflüssigkeiten bediente man sich bisher der Mikropipetten. Bei der Verwendung von Mikropipetten ist es notwendig, daß die Arzneimittelflüssigkeit in größeren Vorratsbehältern, die das Abfüllen der Mikropipetten gestatten, vorliegt. Arzneimittelflüssigkeiten, die sehr leicht kontaminieren oder die in ihrer Herstellung sehr teuer sind (z. B. Interferonlösungen) lassen sich so nicht bereitstellen; sie können nicht z. B. mittels Mikropipetten verabreicht werden.

Durch das deutsche Gebrauchsmuster Nr. 8l 07 209 ist ein aufklappbares Klemmstuck mit Balgaufsatz zur Aufnahme eines langgestreckten zylindrischen Röhrchens bekannt. Nachteilig wirkt sich bei dieser Vorrichtung aus, daß der Inhalt des zylindrischen Röhrchens bei der Entnahmeprozedur leicht in einzelne Kolonnen aufgespalten wird und sich nicht mehr in Form von Tropfen gleichen Volumens durch Druck auf den Balgaufsatz entnehmen läßt.

Erfindungsgemäß lassen sich diese Probleme dadurch lösen, daß ein langgestrecktes Röhrchen, beispielsweise eine Kapillare, welches die Arzneimittelflüssigkeit enthält und an seinen beiden Enden verschlossen ist, in einen röhrenförmigen Applikator, dessen Innendurchmesser dem Durchmesser des Röhrchens angepaßt ist, bündig eingeschoben wird. Beim Einschieben erreicht das vordere Ende des Röhrchens eine konusartig sich verengende Stelle des Innenraumes des röhrenförmigen Applikators, bei weiterem Einschieben des Röhrchens sitzt dessen vorderer Teil schliesslich in dieser konusartigen Verengung fest. Die konusartige Verengung mündet am Ende in einen Zylinderhohlraum. Der Zylinderhohlraum dient zur Aufnahme eines Kolbens, der sich manuell mittels eines in axialer Richtung aus dem Applikator herausragenden Griffes betätigen läßt.

Die Länge des röhrenförmigen Applikators ist so bemessen, daß das in diesen Applikator eingeschobene zylindrische Röhrchen mit seiner Verschlußstelle, z. B. in Form einer Schweißstelle, noch aus dem Ende des Applikators herausragt. Das Hubvolumen des Zylinderhohlraumes muß größer sein als das Volumen des eingeführten, die Flüssigkeit enthaltenden Röhrchens, damit bei der Betätigung des Kolbens eine vollständige Entleerung des Inhalts des nach beiden Seiten geöffneten Röhrchens erfolgen kann.

Die Befüllung des röhrenförmigen Applikators mit einem zylindrischen Röhrchen, das die Arzneimittelflüssigkeit enthält und an seinen beiden Enden z. B. mittels Schweißstellen zunächst noch verschlossen ist, erfolgt in der Weise, daß der Kolben mittels seines Griffes bis zu einem Anschlag herausgezogen wird; jetzt wird das eine Ende des zylindrischen Röhrchens, z. B. nach der Schweißstelle, mit einer Schere aufgeschnitten, das halbseitig geöffnete zylindrische Röhrchen wird mit seinem offenen Ende voran in den Hohlraum des Applikatorröhres bis zum Festanliegen seines vorderen Teiles an dem dafür vorgesehenen Innenkonus eingeschoben, anschließend schneidet man das andere, aus dem Applikator herausragende Ende des zylindrischen Röhrchens, z. B. nach der Schweißstelle, ab; schiebt man jetzt den Kolben zurück, so läßt sich der flüssige Inhalt des zylinderförmigen Röhrchens tropfenweise zur Applikation an dafür vorgesehenen Korperstellen aus dem zylindrischen Röhrchen herausdrücken.

Der Gegenstand der Erfindung soll anhand der Figuren I, 2 und 3 erläutert werden. Die Figur I zeigt, halbseitig, einen Querschnitt des röhrenförmigen Applikators; hierbei ist (I) die Wandung des Applikators, (2) ist der an den Durchmesser des einzuschiebenden Röhrchens angepaßte Innenhohlraum des Applikators; die Position (3) kennzeichnet den Innenkonus des röhrenförmigen Applikators, der ein festes, bündiges Anliegen des eingeschobenen zylindrischen Röhrchens, das die zu applizierende Arzneimittelflüssigkeit enthält, gewährleistet. Der Hohlraum des Innenkonus (3) mündet in den Zylinderhohlraum (4). Die Position (5) zeigt einen Kolben im Zylinderhohlraum, der über einen Schaft (6) mit dem Handgriff (7) verbunden ist. Die Position (8) kennzeichnet einen Anschlag für den Kolben (5). Eine Stützvorrichtung (9), die vorzugsweise in Form eines rundumlaufenden Stützwulstes ausgebildet ist und welche außermittig in Richtung der Applikatorspitze (I0) angebracht ist, verhindert eine Berührung des aufgeschnittenen, in den Applikator eingelegten zylindrischen Röhrchens mit dem Untergrund, z. B. mit der Tischoberfläche, wodurch eine Kontaminierung verhindert wird.

Es ist von Vorteil, wenn der Handgriff (7) polygonal, z. B. in Form eines Dreiecks oder Vierecks, ausgebildet ist, wobei die Ecken etwas über den Rand des Applikators hinausragen. Durch eine derartige Ausgestaltung wird das Wegrollen des Applikators auf der Arbeitsplatte verhindert.

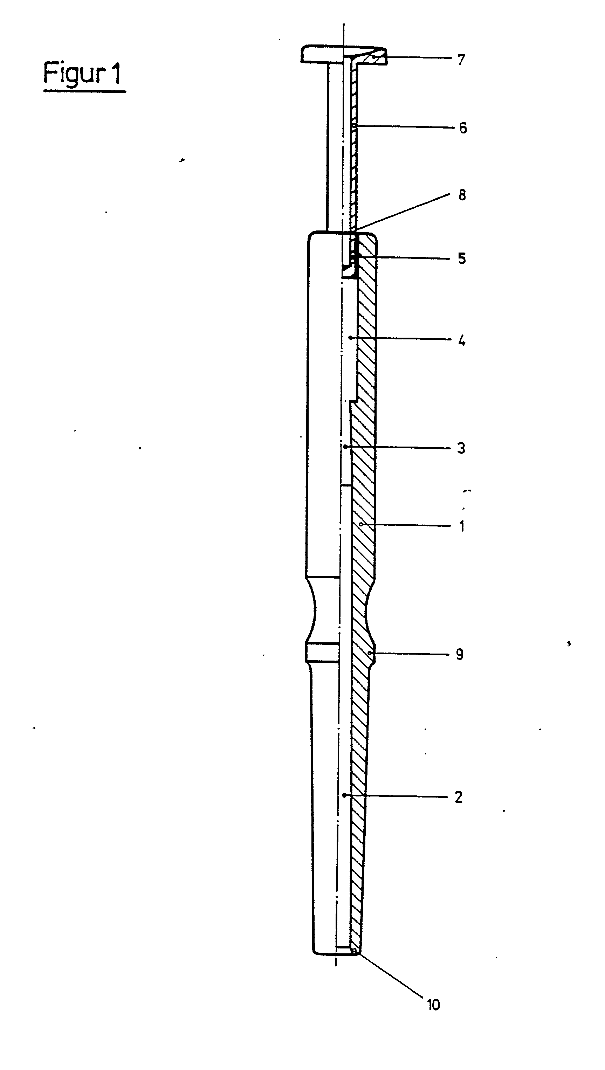

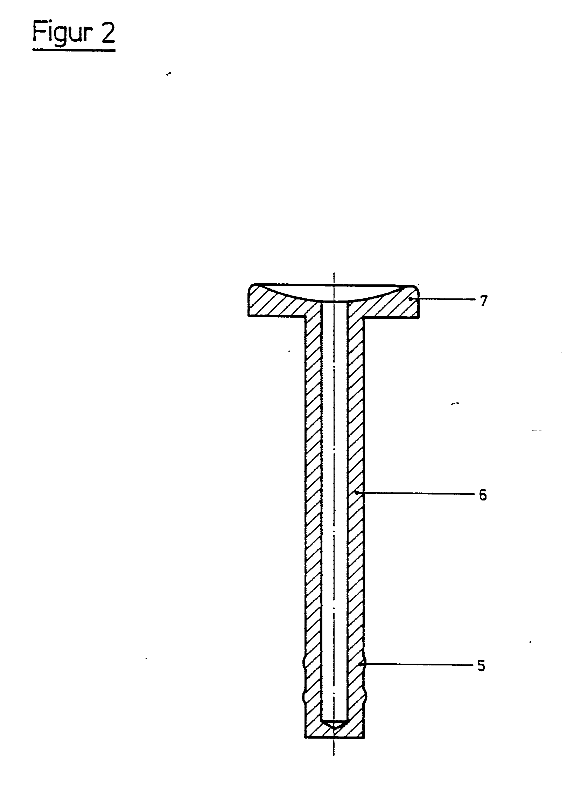

Die Figur 2 zeigt einen Querschnitt durch einen etwas andersartig geformten Kolben (5) mit Schaft (6) und Handgriff (7) zum Einsatz in den Zylinderhohlraum (4) gemäß Figur I.

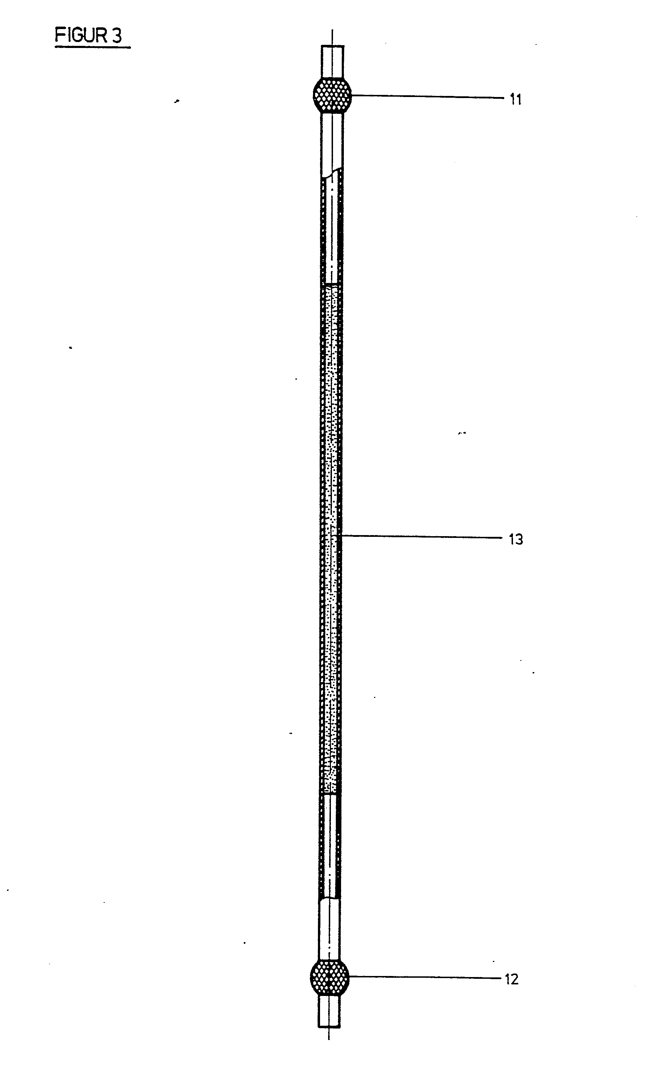

Die Figur 3 zeigt einen Teilschnitt durch ein in den Applikator einzuschiebendes Röhrchen, welches an seinen Enden (II) und (I2) verschweißt ist und welches die Arzneimittelflüssigkeit (I3) enthält.

Der Applikator wird vorzugsweise aus Kunststoffen wie Polyethylen oder Polypropylen gefertigt.

**Ansprüche**

I. Kolbenapplikator mit Klemmvorrichtung zur Aufnahme eines Primärpackmittels in Form eines langgestreckten zylindrischen Röhrchens, dadurch gekennzeichnet, daß er aus einem röhrenförmigen Applikator (I) mit einem an den Durchmesser des zylindrischen Röhrchens angepaßten Innendurchmesser des Innenhohlraumes (2), einem sich an den Innenhohlraum (2) anschließenden Innenkonus (3), einem sich an den Innenhohlraum (3) anschließenden Zylinderhohlraum (4) mit Kolben (5), Schaft (6) und Handgriff (7) und aus einem, den Hub des Kolbens (5) begrenzenden Anschlag (8) besteht und der Applikator außermittig in Richtung der Applikatorspitze (I0) eine Stützvorrichtung (9) aufweist.

2. Vorrichtung nach Anspruch I, dadurch gekennzeichnet, daß die Stützvorrichtung (9) die Form eines rundumlaufenden Stützwulstes aufweist.

3. Vorrichtung nach den Ansprüchen I und 2, dadurch gekennzeichnet, daß der Handgriff (7) polygonal ausgebildet ist und seine Ecken über den Applikatorrand hinausragen.

4. Vorrichtung nach den Ansprüchen I, 2 und 3, dadurch gekennzeichnet, daß das Hubvolumen des Zylinderhohlraumes (4) größer ist als das Volumen des in den Applikator einzulegenden zylindrischen Röhrchens gemäß Figur 3.

Figur 1

Figur 2

FIGUR 3

11

13

12